# EUROPÄISCHE PATENTSCHRIFT

(11) **EP 0 887 100 B1**
(45) Veröffentlichungstag und Bekanntmachung des Hinweises auf die Patenterteilung: **04.01.2006**
(21) Anmeldenummer: 98111760.9
(22) Anmeldetag: 25.06.1998
(51) Int. Cl.: B01D 63/02, B01D 65/00, A61M 1/18

(54) **System bestehend aus Filtervorrichtung und Vorrichtung zu dessen Halterung**
System comprising a filtration device and a support device therefor
Systeme comprenant un dispositif de filtrage et son dispositif de support

(30) Priorität: 26.06.1997 DE 19727251
(43) Veröffentlichungstag der Anmeldung: 30.12.1998
(73) Patentinhaber: Fresenius Medical Care Deutschland GmbH, 61352 Bad Homburg (DE)
(72) Erfinder: Hahmann, Uwe, 66606 St. Wendel (DE); Spengler, Gerhard, 97502 Euerbach (DE); Heilmann, Klaus, 66606 St. Wendel (DE); Wiesen, Gerhard, 66606 St. Wendel (DE); Schmitt, Franz-Jozef, 97422 Schweinfurt (DE); Wunderlich, Ingrid, 09114 Chemnitz (DE)
(74) Vertreter: Gossel, Hans K.

(56) Entgegenhaltungen:
- EP-A- 0 818 228
- WO-A-88/01895
- FR-A- 2 340 758
- JP-A- 10 005 746
- US-A- 4 231 871
- US-A- 5 342 518

## Beschreibung

Die Erfindung betrifft ein System bestehend aus einer Filtervorrichtung und einer Vorrichtung zur Halterung der Filtervorrichtung, sowie die Filtervorrichtung zur Verwendung in einem derartigen System und die Halterung für eine derartige Filtervorrichtung.

Filtervorrichtungen dieser Art, die beispielsweise als Dialysatoren, Hämofilter oder Ultrafilter verwendet werden, sind in unterschiedlichen Ausführungsformen bekannt.

Vorzugsweise als Dialysatoren verwendete Filtervorrichtungen dieser Art sind beispielsweise in EP 0 441 721 B1 und EP 0 525 317 A1 beschrieben worden.

Aus DE-36 41 843 A1 ist eine Hämodialysevorrichtung mit Sterilisiereinrichtung bekannt, bei der Filtervorrichtungen der eingangs angegebenen Art einmal als Dialysator und zum anderen als Sterilfitter für die Dialysierflüssigkeit verwendet werden.

Filtervorrichtungen der eingangs angegebenen Art bestehen üblicherweise aus rohrabschnittförmigen Gehäusen mit Endkappen, die mit zwei axialen und zwei radialen Anschlussstutzen für die durchzuleitenden Flüssigkeiten versehen sind. Die Anschlussstutzen werden durch übliche Verbindungen oder Konnektoren mit Schläuchen und/oder Rohrleitungssystemen verbunden. Diese Anschlüsse lassen sich nicht nur häufig durch umständliche Manipulationen bewirken, sie bedingen zusätzlich auch aufwendige Konnektoren mit einer komplizierten Anschlusstechnik, die die Anwendung verteuern. Darüber hinaus können die Konnektoren auch Ursache für Undichtigkeit sein, was insbesondere bei der Hämodialyse zu schwerwiegenden Gesundheitsschäden führen kann.

Aus der US 5,342,518 ist bereits eine Filtervorrichtung nach dem Oberbegriff des Anspruchs 2 bekannt.

Die nachveröffentlichte und daher nur gemäß Artikel 54(3) EPÜ für die Länder Deutschland, Frankreich, Großbritannien und Italien berücksichtigende EP 0 818 228 A1 beschreibt bereits eine Vorrichtung zur Halterung von Filtervorrichtungen mit Anschlüssen, die in dichtendem Eingriff mit den Filtervorrichtungsanschlüssen in einer ersten Position stehen. Sie sind über Führungsmittel in eine zweite Position verschiebbar, in der die Anschlüsse fluchtend ausgerichtet sind.

Aufgabe der Erfindung ist es, ein System bestehend aus Filtervorrichtung und entsprechender Halterung sowie eine Filtervorrichtung und eine für die Filtervorrichtung vorgesehene Halterung der eingangs angegebenen Art zu schaffen, die einen einfachen und sicheren Anschluss an ein Schlauch- oder Rohrleitungssystem ermöglicht.

Erfindungsgemäß wird diese Aufgabe durch die Merkmalskombination des Anspruchs 1 sowie die Ansprüche 2 bzw. 10 in der Fassung für die Vertragsstaaten Deutschland, Frankreich, Großbritannien und Italien und 10 in der Fassung für die Vertragsstaaten Österreich, Belgien, Schweiz und Liechtenstein, Spanien und Niederlande.

Die erfindungsgemäße Filtervorrichtung lässt sich einfach und schnell mit einer Filter- oder Dialysemaschine oder einem die Filtervorrichtung halternden Gehäuse durch Aufstecken in der Weise verbinden, dass die Anschlüsse oder Anschlussstutzen fluchtend mit Gegenanschlüssen oder Gegenanschlussstutzen ausgerichtet sind und anchließend in dichtender weise zusammengeführt werden Können. Die Anschlüsse oder Anschlussstutzen und deren Gegenteile müssen derart aufeinander abgestimmt sein.

Aus WO88/01895 sind Filtervorrichtungen der eingangs angegebenen Art bekannt, deren auf deren rohrabschnittförmigen Gehäuse aufgesetzten Endkappen mit jeweils zueinander parallelen und mit jeweils einem der beiden Strömungsräume in Verbindung stehenden Durchgängen versehen sind, so daß sich die Filtervorrichfungen durch dichtendes Aneinanderlegen zu Filterbatterien zusammenfassen lassen, in denen die einzelnen Filtervorrichtungen parallel zueinander geschaltet sind. Die Endkappen der Filtervorrichtungen mit den fluchtenden Durchgangsbohrungen sind durch Schraubbolzen miteinander verspannt, wobei auf die außen liegenden Kappenseiten Verschlußplatten aufgesetzt sind, die Anschlußstutzen tragen, soweit die betreffenden Enden der Durchgangsbohrungen als Ein- oder Auslässe dienen sollen.

Die Anschlüsse können rohrabschnittförmige Mündungen aufweisen.

Die Mündungen der Anschlüsse können in zueinander parallelen Ebenen oder aber auch in einer gemeinsamen Ebene liegen.

Die Mittellinien der Mündungen liegen zweckmäßigerweise auf einer gemeinsamen Längsebene, die die Mittellinie des Gehäuses enthalten oder parallel zu der Mittellinie des Gehäuses verlaufen kann.

Die Mündungen können auch auf zueinander parallelen Ebenen liegen, die radial zu dem Gehäuse verlaufen.

Nach einer bevorzugten Ausführungsform ist vorgesehen, daß sich die Anschlüsse an den Endkappen des Gehäuses befinden. Bei dieser Ausführungsform ist das Gehäuse selbst, das zweckmäßigerweise aus einem Rohrabschnitt besteht, frei von Anschlüssen, so daß die Gehäusewandung dünnwandiger und damit materialsparend ausgeführt werden kann. Befinden sich die Anschlüsse oder Anschlußstutzen an den Endkappen, können diese robuster ausgeführt werden, ohne das Gehäuse selbst zu belasten.

Nach einer bevorzugten Ausführungsform ist vorgesehen, daß die Endkappen mit radialen, über deren Böden verlaufenden Wülsten versehen sind, in deren die Kappenränder überragenden Enden die Anschlüsse münden. Auf diese Weise ist eine besonders stabile Bauweise gegeben, die den zwischen den Kappen eingefaßten Gehäuseteil frei von Anschlüssen und durch diese bedingten Spannungen hält.

Die Erfindung betrifft weiterhin eine Vorrichtung zur Halterung von Filtervorrichtungen mit radialen Anschlüssen, vorzugsweise von Filtervorrichtungen der vorstehend beschriebenen Art.

Filtervorrichtungen, die üblicherweise nur zu einem einmaligen Gebrauch bestimmt sind, müssen sich einfach, schnell und sicher mit einer Filter- oder Dialysemaschine verbinden und einfach und schnell austauschen lassen.

Eine Vorrichtung zur Halterung von Filtervorrichtungen mit seitlichen Anschlüssen, deren Mittellinien parallel zueinander laufen, vorzugsweise von Filtervorrichtungen der vorstehend beschriebenen Art, ist durch eine Einrichtung gekennzeichnet, mit der sich die Filtervorrichtung in einer Stellung zu der Halterung positionieren läßt, in der deren Anschlüsse oder Anschlußstutzen fluchtend zu den Tüllen der Halterung ausgerichtet sind, und mit der sich nach entsprechender Ausrichtung die Anschlüsse oder Anschlußstutzen dichtend auf die Tüllen aufschieben oder aufdrücken lassen. Die Einrichtung kann aus einer Führung mit zwei Führungsabschnitten bestehen, von denen der erste dem Aufsetzen und Positionieren der Filtervorrichtung dient und der zweite dem lagerichtigen Aufschieben der Filtervorrichtung, so daß die Anschlüsse oder Anschlußstutzen der Filtervorrichtung mit dichtendem Kontakt auf die Tüllen aufgeschoben werden.

Die Einrichtung kann auch aus einer Schwenklagerung für die Filtervorrichtung bestehen, in die diese zu ihrer Positionierung eingehängt wird, so daß die Filtervorrichtung nach dem Einhängen lagerichtig mit ihren Anschlußstutzen auf die Tüllen der Halterung aufgedrückt werden kann.

Die Einrichtung kann auch aus einer mit einer Betätigungseinrichtung versehenen Führungseinrichtung bestehen, in die die Filtervorrichtung eingesetzt wird und die anschließend die Filtervorrichtung derartig in Richtung auf die Halterung schiebt oder drückt, daß deren Anschlüsse oder Anschlußstutzen dichtenden Eingriff mit den Tüllen kommen.

Eine bevorzugte Ausführungsform ist durch ein längliches rahmen- oder gehäuseförmiges Halteteil gekennzeichnet, das mit Führungen zur Aufnahme von Gegenführungsstücken der Filtervorrichtungen zu deren Ausrichtung in deren Querrichtung und deren Tiefe und mit einem Anschlag zu deren Ausrichtung in deren Längsrichtung versehen ist, wobei ausfahrbare Tüllen vorgesehen sind, die in dem ausgerichteten Zustand einer aufgesetzten Filtervorrichtung in dichtend in deren Stutzen eingreifen.

Zweckmäßigerweise bestehen die Führungen aus Nuten, deren Flanken mit zu deren Rändern parallelen Nuten oder Hinterschlitten versehen sind, in die zueinander parallele Rippen greifen, die als Gegenführungsstücke seitlich der Anschlüsse oder Stutzen der Filtervorrichtungen angeordnet sind.

Die Flanken oder Ränder der oberen Nut können mit dem Anschlag versehen sein.

Nach einer besonders bevorzugten Ausführungsform ist vorgesehen, daß die Tüllen an einem Gleitstück angeordnet sind, das in Richtung von deren Längsachsen in dem Halteteil verschieblich geführt ist, daß das Gleitstück in Richtung auf die aufgesetzte Filtervorrichtung durch mindestens ein federndes Element belastet ist und daß eine das federnde Element spannende Einrichtung vorgesehen ist, durch deren Lösen die Tüllen schnappend in die Anschlüsse oder Stutzen der aufgesetzten Filtervorrichtung eingefahren werden. Diese Ausgestaltung der Erfindung stellt sicher, daß nach dem Aufsetzen und zentrieren der Filtervorrichtung auf dem Halteteil einfach und schnell die Verbindung von den Anschlüssen oder Stutzen der Filtervorrichtung zu den Tüllen und damit zu der Filter- oder Dialysemaschine hergestellt werden können.

Die das federnde Element spannende Einrichtung kann aus einem Kniehebel oder einem Exzenter bestehen, der in seiner Spannstellung in seine Totpunktstellung bewegt worden ist. Wird der Exzenter oder der Kniehebel aus seiner Totpunktstellung gelöst, greifen die Tüllen schnappend in die Stutzen der aufgesetzten Filtervorrichtung ein.

Nach einer weiteren bevorzugten Ausführungsform ist vorgesehen, daß das die Tüllen tragende Gleitstück einer Seite in einem Schlitten gelagert ist, der zur Anpassung an unterschiedliche Abstände der endseitigen Anschlüsse oder Stutzen der Filtervorrichtung in dem Halteteil begrenzt verschieblich geführt ist.

Das Gleitstück kann zur Ausrichtung des Schlittens mit einer Positioniereinrichtung versehen sein, die an einem den Schlitten positionierenden Anschlag der Filtervorrichtung angreift.

Die Positioniereinrichtung besteht zweckmäßigerweise aus einem Zapfen mit einer Schrägfläche, die an einer Anschlagkante der Filtervorrichtung angreift.

Ein Ausführungsbeispiel der Erfindung wird nachstehend anhand der Zeichnung näher erläutert. In dieser zeigt
- Fig. 1: eine Ansicht der Seite der Filtervorrichtung, in der deren Anschlüsse münden,
- Fig. 2: einen Schnitt durch die Filtervorrichtung längs der Linie A-A in Fig. 1,
- Fig. 3: eine um 90° gedrehte Seitenansicht der Filtervorrichtung nach Fig. 1,
- Fig. 4: eine Draufsicht auf die Filtervorrichtung nach den Figuren 1 bis 3,
- Fig. 5: eine perspektivische Ansicht des Gehäuses der Filtervorrichtung mit abgehobenen Endkappen,
- Fig. 6: eine perspektivische Ansicht der in ein Halteteil einer Filter- oder Dialysemaschine eingesetzten Filtervorrichtung nach den Fig. 1 bis 5,
- Fig. 7: eine perspektivische Ansicht des Halteteils nach Fig. 6 ohne die in diesem gehalterte Filtervorrichtung,
- Fig. 8: eine Rückansicht des Halteteils nach den Fig. 6 und 7,
- Fig. 9: einen Schnitt durch das Halteteil längs der Linie A-A in Fig. 8,
- Fig. 10: einen Schnitt durch das Halteteil längs der Linie B-B in Fig. 8,
- Fig. 11: einen Schnitt durch das Halteteil längs der Linie C-C in Fig. 8,
- Fig. 12: einen Schnitt durch das Halteteil längs der Linie D-D in Fig. 8,

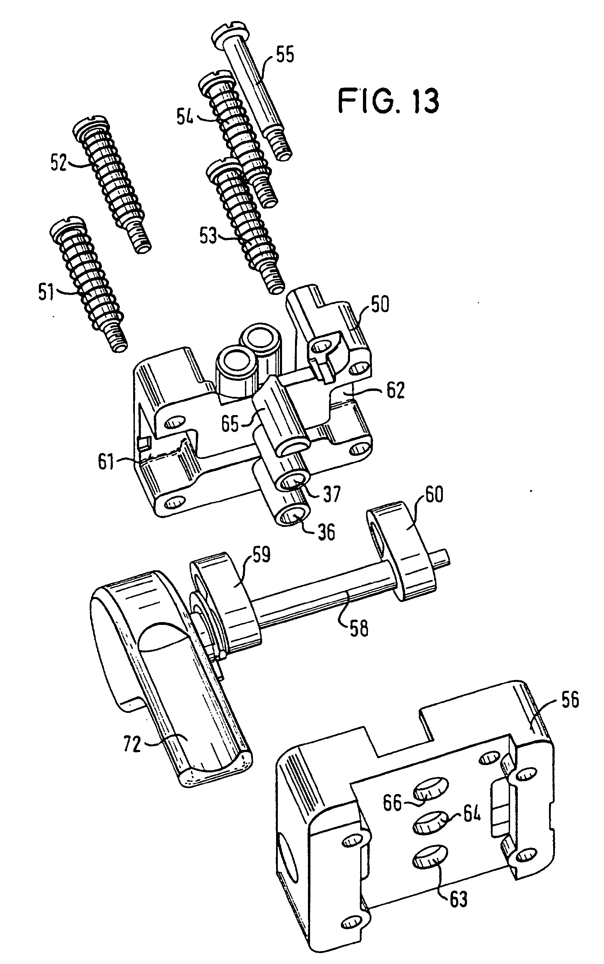

- Fig. 13: den die unteren Tüllen tragenden Schlitten des Halteteils im auseinandergezogenen Zustand seiner Einzelteile.

Die Filtervorrichtung besteht aus einem rohrförmigen Gehäuse 1, dessen Enden durch glockenförmige Randbereiche 2 in ihren Durchmessern vergrößert sind. An der Innenseite des sich glockenförmig erweiternden Randes 2 schließen sich frei auskragende, leistenförmige Fortsätze 3 an, die über den Umfang verteilt etwa gleiche Abstände voneinander aufweisen und mit der zylindrischen Wandung des Gehäuses 1 fluchten. Diese leistenförmigen Fortsätze dienen der Halterung der die Enden des nicht dargestellten Faserbündels einbindenden Vergußmassen.

Auf die ringförmigen Stirnflächen der sich glockenartig erweiternden Ränder des Gehäuses 1 stoßen stumpf die Stimflächen der zylindrischen Ränder 4 der etwa becherförmigen Endkappen 5. Die Stimflächen der Endkappen 4 und der Ränder 2 des Gehäuses 1 sind miteinander dichtend verklebt oder verschweißt.

Auf die nach außen gewölbten Böden 6 der Endkappen 5 sind radial verlaufende, nasenförmige Wülste 7 aufgesetzt, die die Kappen 5 nach einer Seite hin überragen. In den die Endkappen 5 radial überragenden Enden der Wülste münden jeweils zwei Anschlüsse 8, 9 und 10, 11, deren Mündungen in einer gemeinsamen, zu der Mittellinie des Gehäuses 1 parallelen Ebene liegen. Die jeweils außen liegenden Anschlüsse 8, 11 stehen mit den nicht dargestellten Faserhohlräumen in Verbindung, während die inneren Anschlüsse 9, 10 in den das Faserbündel einfassenden Raum des Gehäuses münden.

Die Mündungen der Anschlüsse 8 bis 11 sind rahmenartig von der Stirnseite der Wülste 12, 13 eingefaßt, die an ihren äußeren Seiten mit nach außen ragenden, zueinander parallelen Rippen 14, 15 versehen sind, die der Verankerung an einem Halteteil eines Gehäuse dienen, das mit Gegenanschlüssen für die Anschlüsse 8 bis 11 versehen ist, wobei die Konnektierung der Anschlüsse durch einfaches Aufstekken oder Aufschieben bewirkt werden kann.

Das Gehäuse 1 und die Kappen 5 bestehen in üblicher Weise aus Kunststoffspritzgußteilen.

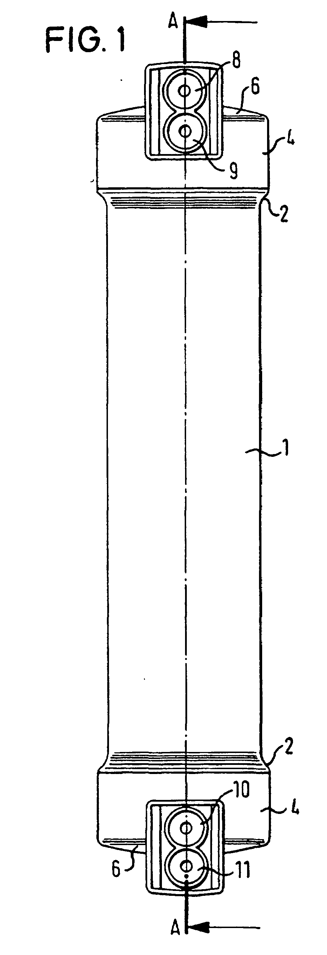

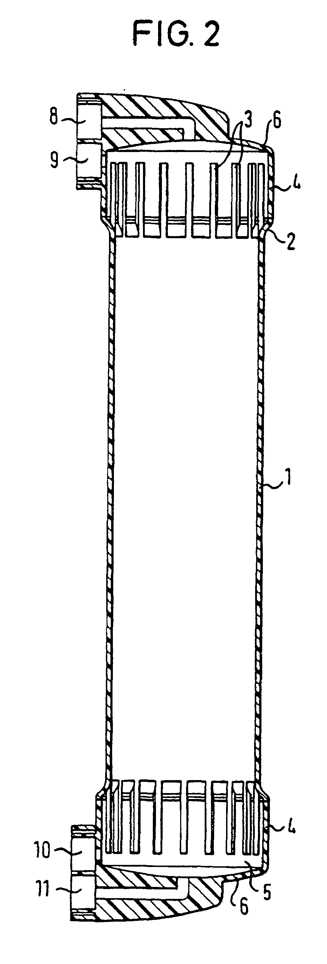

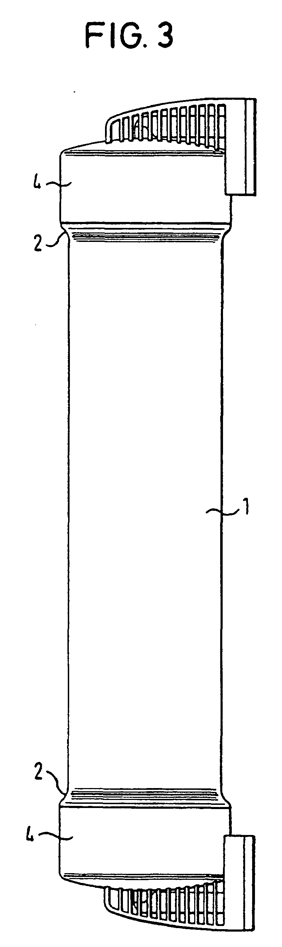

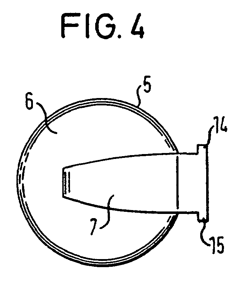

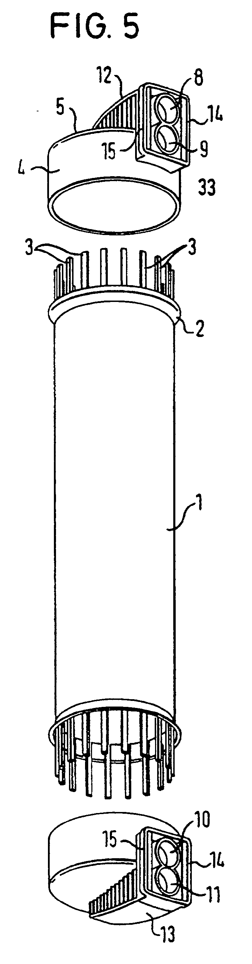

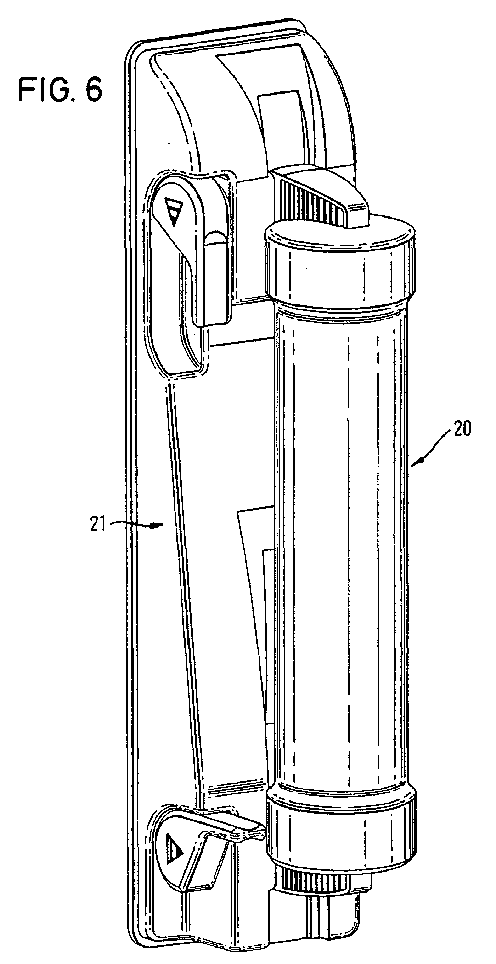

Aus Fig. 6 ist ein im ganzen als 21 bezeichnetes Halteteil ersichtlich, das in nicht dargestellter Weise an dem Gehäuse einer Filter- oder Dialysemaschine befestigt ist. In das Halteteil 21 ist die im ganzen mit 20 bezeichnete Filtervorrichtung der anhand der Fig. 1 bis 5 beschriebenen Art in der Weise eingesetzt, daß deren Anschlüsse 8 bis 9 mit ausfahrbaren Tüllen des Halteteils 21 dicht verbunden sind.

Das Halteteil 21 besteht aus einem schalenförmigen Gehäuse 22, dessen offene Seite mit dem Gehäuse einer Filter- oder Dialysevorrichtung in der Weise verbunden ist, daß deren umlaufender Rand an einer Außenseite einer Gehäusewand anliegt.

Das schalenförmige Gehäuse weist eine mittlere Einziehung 23 auf, so daß obere und untere Gehäuseteile 24, 25 vorspringen und den eingezogenen mittleren Gehäuseteil 23 einfassen.

In den vorspringenden oberen und unteren Gehäuseteilen 24, 25 sind fluchtende Nuten 26, 27 angeordnet, die nach oben hin in sich trichterförmig erweiternder Form frei auslaufen. Die fluchtenden Nuten 26, 27 sind in ihren einander gegenüberliegenden Flanken mit weiteren Nuten 28, 29 versehen, die nach außen hin durch Randstege 30, 31 begrenzt sind. Die oberen Randstege 30, 31 sind mit stufenförmigen Anschlägen 32 versehen.

Die Breite der nasenförmigen Wülste 7 der Filtervorrichtungen 20 unmittelbar vor den die äußeren Ränder bildenden Rippen 14, 15 entspricht dem Abstand zwischen den Randleisten 30, 31 der Nuten 26, 27, so daß sich die Filtervorrichtung von oben her über die sich trichterförmig verbreiternden Einläufe in die Nuten 26, 27 des Halteteils in der Weise einsetzen läßt, daß die Rippen in den in den Flanken vorgesehenen Nuten 28, 29 geführt werden und die Randstege 30, 31 der Nuten 26, 27 hintergreifen. Dabei lassen sich die rahmenartigen Stirnseiten der Wülste 12, 13 soweit in die Nuten 26, 27 des Halteteils 21 einschieben, bis die Unterkante 33 der rahmenförmigen Einfassung der Stirnseite der oberen Wulst 12 auf die in den Leisten 30, 31 gebildeten Anschläge 32 stößt. In dieser Stellung sind die oberen beiden Tüllen 34, 35 zu den oberen Anschlüssen 8, 9 ausgerichtet, so daß diese Tüllen in die Anschlüsse eingefahren werden können.

Die unteren Tüllen 36, 37 werden nach entsprechender Ausrichtung in die unteren Anschlüsse 10, 11 eingefahren.

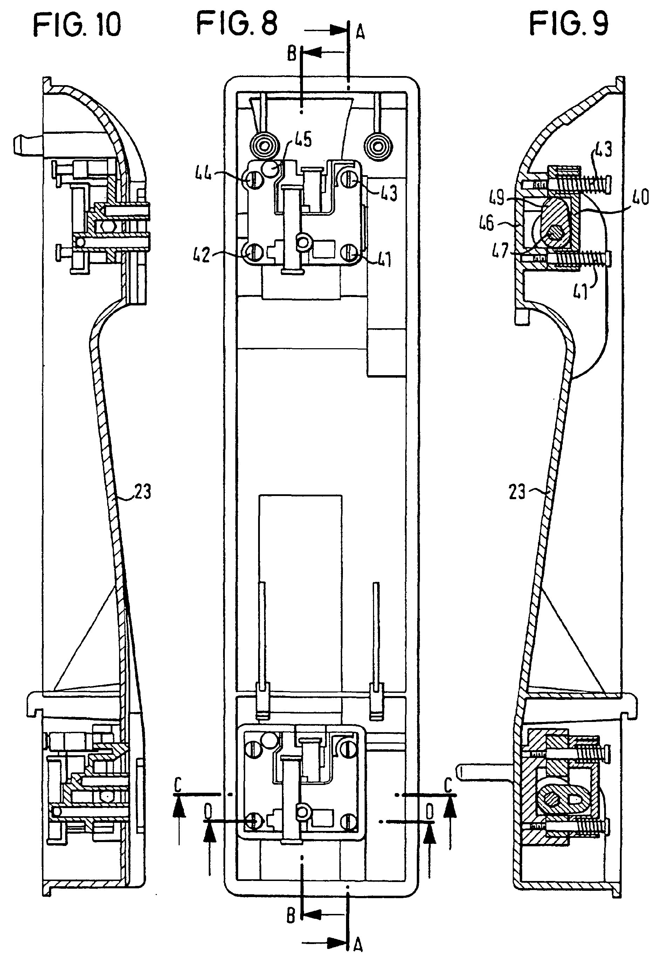

Die oberen Tüllen 34, 35 sind an einem Gleitstück 40 angeordnet, das auf fünf Zapfen 41 bis 45 längsverschieblich geführt ist. Die Zapfen 41 bis 45 sind in Bohrungen des Gehäuses eingeschraubt. Die Zapfen 41 bis 44 sind mit verbreiterten Köpfen versehen, auf denen sich die einen Enden von Druckfedern abstützen, deren anderen Enden an dem Gleitstück 40 anliegen und dieses in Richtung der Gehäusewandung 46 beaufschlagen. In den Seitenwänden des Gehäuses 21 ist eine Welle 47 drehbar gelagert, die einen außen liegenden Hebel 48 trägt. In dem Gehäuse ist auf der Welle 47 ein Exzenter 49 befestigt, der sich mit seiner exentrischen Fläche auf der benachbarten Wand des Gleitstücks 40 in der aus Fig. 9 ersichtlichen Weise abwälzen kann. Durch den Hebel 48 läßt sich der Exzenter 49 in seine Totpunktstellung bzw. über Totpunktstellung drehen, in der das Gleitstück auf den Bolzen 41 bis 45 nach innen verschoben wird, so daß die Federn noch stärker vorgespannt werden. In dieser vorgespannten Form wird die Filtervorrichtung 20 in die Nuten 26, 27 eingesetzt. Wird nun der Hebel 48 aus seiner aufrechten Stellung einwärts geschwenkt, schnappt der Exzenter 49 in seine aus Fig. 9 ersichtliche Stellung, so daß die Druckfedern das Gleitstück 40 nach außen verschieben und die Tüllen 34, 35 dichtend mit den Anschlüssen oder Stutzen 8, 9 verbinden, so daß die Tüllen 34, 35 mit den Stutzen 8, 9 konnektiert sind.

Der Zapfen 45, der nicht von einer Druckfeder eingefaßt ist, dient der Führung des Gleitstücks 40 in dem Gehäuse 21.

Die Tüllen 36, 37 sind auf einem Gleitstück 50 angeordnet, das auf den Bolzen 51 bis 55 geführt ist, deren vorderen Enden in den Klotz 56 eingeschraubt sind, wobei die Bolzen 51 bis 54 Druckfedern einfassen, die sich einerseits auf den verbreiterten Köpfen der Bolzen und andererseits auf dem Gleitstück 50 abstützen. Der weitere Bolzen 55 dient ausschließlich der Führung des Gleitstücks 50.

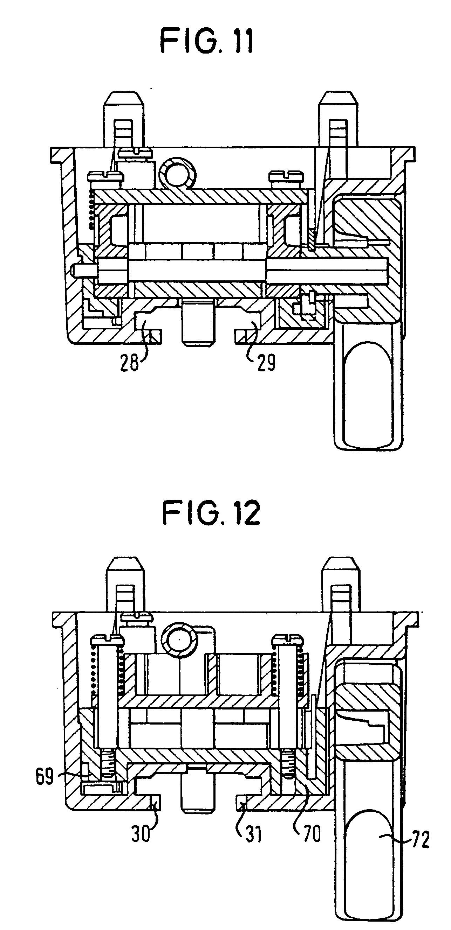

In den Seitenwänden des Klotzes 56 ist eine Welle 58 gelagert, auf der zwei Exzenter 59, 60 befestigt sind, die sich in der aus Fig. 11 ersichtlichen Weise auf Deckwandungen 61, 62 des Gleitstücks 50 abstützen können, die seitliche Aussparungen des Gleitstücks 50 begrenzen. Die mit dem Gleitstück 50 verbundenen Tüllen 36, 37 durchsetzen Bohrungen 63, 64 des Klotzes 36.

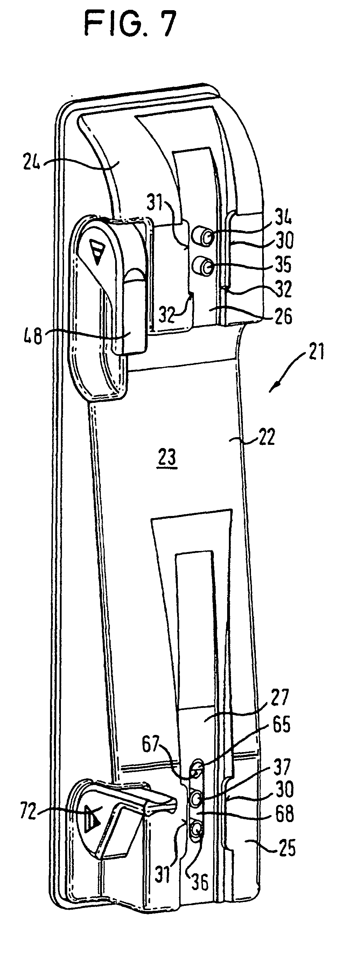

Das Gleitstück 50 ist in Reihe mit den Tüllen mit einem Zentrierstift 65 versehen, der den Klotz 56 in einer weiteren Bohrung 66 durchsetzt. Der Zentrierstift 65 ist in der aus Fig. 7 ersichtlichen Weise mit einer Abschrägung versehen.

Die Tüllen 36 und 37 sowie der Zentrierstift 65 durchsetzen den Grund der Nut 27 in einem aus Fig. 7 ersichtlichen Langloch 68, in dem diese begrenzt verschieblich sind.

Der Klotz 56 ist in der aus den Fig. 11 und 12 ersichtlichen Weise mit seitlichen Kufen 69, 70 versehen, so daß er in Längsrichtung des Gehäuses 21 in komplementären Führungsnuten begrenzt verschieblich ist.

Die die Exzenter 59, 60 tragende Welle 58 ist auf der Außenseite des Gehäuses 22 mit einem Hebel 72 versehen, durch den die Exzenterwelle verdrehbar ist.

Vor dem Aufsetzen des Filtermoduls 20 in die Nuten 26, 27 des Gehäuses 21 werden die beiden Hebel 48 und 72 senkrecht gestellt, so daß die Exzenter die Gleitstücke 40 und 50 in der beschriebenen Weise vorspannen und sich die Tüllen in ihrer eingezogenen Stellung befinden. Anschließend wird das Filtermodul 20 in die Nuten eingesetzt, bis sich der untere Rand 33 der rahmenförmigen Stirnseite der Wulst 12 auf den Anschlägen 32 abstützt. Zur Konnektierung der Tüllen 34, 35 mit den Anschlüssen 8, 9 wird dann der Hebel 48 in der aus Fig. 7 ersichtlichen Weise umgelegt, so daß die Tüllen 34, 35 schnappend und dichtend in die Anschlüsse 8, 9 einfahren.

Anschließend wird auch der untere Hebel 72 umgelegt, so daß das untere Gleitstück ausfährt und die Tüllen 36, 37 schnappend in die Anschlüsse 10, 11 des Filtermoduls einfahren. Sollte aufgrund von Fertigungstoleranzen der Abstand der Tüllen zu den Anschlüssen 10, 11 nicht genau stimmen, folgt eine Ausrichtung durch den Justierstift 65, dessen Schrägfläche an den oberen Rand der rahmenförmigen Einfassung der Stirnseite der unteren Wulst 13 des Filtermoduls angreift und über seine Schrägfläche 67 den schlittenförmigen Klotz 56 soweit anhebt, daß die Tüllen 36, 37 fluchtend in die Ausnehmungen 10, 11 einfahren können.

Zur Befestigung in einer Gehäusewand der Filter- oder Dialysemaschine ist das schalenförmige Gehäuse 21 auf seiner Rückseite mit Füßen versehen, die zur Verbindung Rastausnehmungen aufweisen.

Das schalenförmige Gehäuse 21 und die Gleitstücke sowie der schlittenartige Klotz 56 können aus Kunststoffspritzgußteilen bestehen.

## Patentansprüche (Patentansprüche für folgende(n) Vertragsstaat(en): AT, BE, CH, LI, ES, NL)

1. System bestehend aus
einer Filtervorrichtung mit einem länglichen Gehäuse (1) mit zwei durch Membranen voneinander getrennten Strömungsräumen mit mindestens einem radial verlaufenden Anschluss (8 - 11) oder Anschlussstutzen, vorzugsweise zwei Anschlüssen oder Anschlussstutzen, für jeden Strömungsraum, wobei die Anschlüsse (8 - 11) auf einer Seite des Gehäuses (1) angeordnet sind und deren Mittellinien parallel zueinander verlaufen, wobei seitlich der Anschlüsse (8 - 11) mit einem Anschlag (33) versehene Gegenführungsstücke angeordnet sind, geeignet zur Ausrichtung der Filtervorrichtung in Längs- und Querrichtung sowie in der Tiefe in Führungen (26, 27) eines Halteteils (21), wobei die Gegenführungsstücke nach außen ragende, zueinander parallele Rippen (14, 15) umfassen, und
einer Vorrichtung zur Halterung der Filtervorrichtung, wobei die Vorrichtung zur Halterung Tüllen (34, 35; 36, 37) aufweist, die mit den Anschlüssen (8 - 11) oder Anschlussstutzen der Filtervorrichtung in dichtenden Eingriff zu bringen sind, und dass eine Einrichtung vorgesehen ist, mit der sich die Filtervorrichtung in einer Stellung zu der Halterung positionieren lässt, in der deren Anschlüsse (8 - 11) oder Anschlussstutzen fluchtend zu den Tüllen (34, 35; 36, 37) der Halterung ausgerichtet sind, wobei die Einrichtung mit Führungen (26, 27) zur Aufnahme von Gegenführungsstücken (14, 15) der Filtervorrichtungen (20) zu deren Ausrichtung in deren Querrichtung und in deren Tiefe und mit einem Anschlag (32) zu deren Ausrichtung in deren Längsrichtung versehen ist, die ein anschließendes Zusammenfügen der Anschlüsse (8-11) und Anschlussstuzten mit den Tüllen (34, 35; 36, 37) in der Weise gewährleisten, dass diese in dichtenden Eingriff miteinander kommen.

2. Filtervorrichtung zur Verwendung in einem System nach Anspruch 1 mit einem länglichen Gehäuse (1)
mit zwei durch Membranen voneinander getrennten Strömungsräumen mit mindestens einem radial verlaufenden Anschluss (8 - 11) oder Anschlussstutzen, vorzugsweise zwei Anschlüssen oder Anschlussstutzen, für jeden Strömungsraum, wobei die Anschlüsse (8 - 11) auf einer Seite des Gehäuses (1) angeordnet sind und deren Mittellinien parallel zueinander verlaufen,
**dadurch gekennzeichnet,**
**dass** seitlich der Anschlüsse (8 - 11) mit einem Anschlag (33) versehene Gegenführungsstücke angeordnet sind, geeignet zur Ausrichtung der Filtervorrichtung in Längs- und Querrichtung sowie in der Tiefe in Führungen (26, 27) eines Halteteils (21), wobei die Gegenführungsstücke nach außen ragende, zueinander parallele Rippen (14, 15) umfassen.

3. Filtervorrichtung nach Anspruch 2, **dadurch gekennzeichnet, dass** die getrennten Strömungsräume durch ein in dem Gehäuse (1) gehaltertes Hohlfaserbündel gebildet werden, bei dem die Faserhohlräume den einen und der das Faserbündel umgebende Raum den anderen Strömungsraum bilden.

4. Filtervorrichtung nach Anspruch 2 oder 2, **dadurch gekennzeichnet, dass** die Anschlüsse (8-11) rohrabschnittförmige Mündungen aufweisen.

5. Filtervorrichtung nach Anspruch 2 bis 4, **dadurch gekennzeichnet, dass** die Mündungen der Anschlüsse (8 - 11) in zueinander parallelen Ebenen liegen.

6. Filtervorrichtung nach einem der Ansprüche 1 bis 5, **dadurch gekennzeichnet, dass** die Mündungen der Anschlüsse (8 - 11) in einer gemeinsamen Ebene liegen.

7. Filtervorrichtung nach einem der Ansprüche 1 bis 6, **dadurch gekennzeichnet, dass** die Mittellinien der Anschlüsse (8 - 11) auf einer gemeinsamen Ebene liegen.

8. Filtervorrichtung nach einem Ansprüche 1 bis 6, **dadurch gekennzeichnet, dass** die Mittellinien der Anschlüsse (8 - 11) auf zueinander parallelen Ebenen liegen.

9. Filtervorrichtung nach einem der Ansprüche 1 bis 8, **dadurch gekennzeichnet, dass** die Filtervorrichtung Endkappen (5) aufweist und dass sich die Anschlüsse (8 -11) an den Endkappen (5) befinden.

10. Filtervorrichtung nach einem der Ansprüche 1 bis 9, **dadurch gekennzeichnet, dass** die Filtervorrichtung Endkappen (5) aufweist, die becherförmig ausgeführt sind und die mit jeweils einer radial über deren Böden (6) verlaufenden Wulst (7) versehen sind, in deren die Kappenränder (5) überragenden Enden die Anschlüsse (8 - 11) münden.

11. Vorrichtung zur Halterung einer Filtervorrichtung gemäß einem der Ansprüche 1 bis 10, **dadurch gekennzeichnet, dass** die Vorrichtung zur Halterung Tüllen (34, 35; 36, 37) aufweist, die mit den Anschlüssen (8 - 11) oder Anschlussstutzen der Filtervorrichtung in dichtenden Eingriff zu bringen sind, und dass eine Einrichtung vorgesehen ist, mit der sich die Filtervorrichtung in einer Stellung zu der Halterung positionieren lässt, in der deren Anschlüsse (8 - 11) oder Anschlussstutzen fluchtend zu den Tüllen (34, 35; 36, 37) der Halterung ausgerichtet sind, wobei die Einrichtung mit Führungen (26, 27) zur Aufnahme von Gegenführungsstücken (14, 15) der Filtervorrichtungen (20) zu deren Ausrichtung in deren Querrichtung und in deren Tiefe und mit einem Anschlag (32) zu deren Ausrichtung in deren Längsrichtung versehen ist, die ein anschließendes Zusammenfügen der Anschlüsse (8 -11) und Anschlussstuzten mit den Tüllen (34, 35; 36, 37) in der Weise gewährleisten, dass diese in dichtenden Eingriff miteinander kommen.

12. Vorrichtung nach Anspruch 11, **dadurch gekennzeichnet, dass** die Führungen (26,27) in einem länglichen rahmen- und gehäuseförmigen Halteteil (21) angeordnet sind und dass die Tüllen (34, 35; 36, 37) ausfahrbar sind und in dem ausgerichteten Zustand einer aufgesetzten Filtervorrichtung (20) dichtend in deren Anschlüssen (8 - 11) oder Stutzen eingreifen.

13. Vorrichtung nach Anspruch 12, **dadurch gekennzeichnet, dass** die Führungen aus Nuten (26, 27) bestehen, deren Flanken mit zu deren Rändern parallelen Nuten (28, 29) versehen sind, in die zueinander parallele Rippen (14, 15) greifen, die als Gegenführungsstücke seitlich der Anschlüsse oder Stutzen (8 - 11) der Filtervorrichtung (20) angeordnet sind.

14. Vorrichtung nach Anspruch 12 oder 13, **dadurch gekennzeichnet, dass** das Halteteil (21) in unterschiedlicher vertikaler Position angeordnete Nuten (26, 27) aufweist und dass die Flanken oder Ränder der oberen Nut (26) mit dem Anschlag (32) versehen sind.

15. Vorrichtung nach einem der Ansprüche 12 bis 14, **dadurch gekennzeichnet, dass** die Tüllen (34, 35; 36, 36) an einem Gleitstück (40, 50) angeordnet sind, das in Richtung von deren Längsachsen in dem Halteteil (21) verschieblich geführt ist, dass das Gleitstück (40, 50) in Richtung auf die aufgesetzte Filtervorrichtung durch mindestens ein federndes Element belastet ist und dass eine das federnde Element spannende Einrichtung vorgesehen ist, durch deren Lösen die Tüllen schnappend in die Anschlüsse oder Stutzen der aufgesetzten Filtervorrichtung eingefahren werden.

16. Vorrichtung nach einem der Ansprüche 12 bis 15, **dadurch gekennzeichnet, dass** die das federnde Element spannende Einrichtung aus einem Kniehebel oder einem Exzenter besteht, der in seiner Spannstellung in seine Totpunktstellung bewegt worden ist.

17. Vorrichtung nach einem der Ansprüche 12 bis 16, **dadurch gekennzeichnet, dass** das die Tüllen tragende Gleitstück (50) einer Seite in einem schlittenartigen Klotz (56) gelagert ist, der zur Anpassung an unterschiedliche Abstände der endseitigen Anschlüsse oder Stutzen (8 - 11) der Filtervorrichtung in dem Halteteil (21) begrenzt verschieblich geführt ist.

18. Vorrichtung nach einem der Ansprüche 12 bis 17, **dadurch gekennzeichnet, dass** das Gleitstück (50) zur Ausrichtung des schlittenartigen Klotzes (56) mit einer Positioniereinrichtung (65) versehen ist.

19. Vorrichtung nach Anspruch 18, **dadurch gekennzeichnet, dass** die Positioniereinrichtung aus einem Zapfen (65) mit einer Schrägfläche (67) besteht, die an einer Anschlagkante der Filtervorrichtung (20) angreift.

## Patentansprüche (Patentansprüche für folgende(n) Vertragsstaat(en): DE, FR, GB, IT)

1. System bestehend aus
einer Filtervorrichtung mit einem länglichen Gehäuse (1) mit zwei durch Membranen voneinander getrennten Strömungsräumen mit mindestens einem radial verlaufenden Anschluss (8 - 11) oder Anschlussstutzen, vorzugsweise zwei Anschlüssen oder Anschlussstutzen, für jeden Strömungsraum, wobei die Anschlüsse (8 - 11) auf einer Seite des Gehäuses (1) angeordnet sind und deren Mittellinien parallel zueinander verlaufen, wobei seitlich der Anschlüsse (8 - 11) mit einem Anschlag (33) versehene Gegenführungsstücke angeordnet sind, geeignet zur Ausrichtung der Filtervorrichtung in Längs- und Querrichtung sowie in der Tiefe in Führungen (26, 27) eines Halteteils (21), wobei die Gegenführungsstücke nach außen ragende, zueinander parallele Rippen (14, 15) umfassen, und
einer Vorrichtung zur Halterung dieser Filtervorrichtung, mit Tüllen (34, 35; 36, 37), die mit den Anschlüssen (8-11) oder Anschlussstutzen der Filtervorrichtung in dichtenden Eingriff zu bringen sind, sowie mit einer Einrichtung, mit der sich die Filtervorrichtung in einer Stellung zu der Vorrichtung zur Halterung positionieren lässt, in der deren Anschlüsse (8 - 11) oder Anschlussstutzen fluchtend zu den Tüllen (34, 35; 36, 37) der Vorrichtung zur Halterung ausgerichtet sind, wobei die Einrichtung mit Führungsmitteln versehen ist, die ein anschließendes Zusammenfügen der Anschlüsse (8 -11) und Anschlussstutzen mit den Tüllen (34, 35; 36, 37) in der Weise gewährleisten, dass diese in dichtenden Eingriff miteinander kommen, wobei ein längliches rahmen- oder gehäuseförmiges Halteteil (21), das mit Führungen (26, 27) zur Aufnahme von Gegenführungsstücken der Filtervorrichtungen (20) zu deren Ausrichtung in deren Querrichtung und in deren Tiefe und mit einem Anschlag (32) zu deren Ausrichtung in deren Längsrichtung versehen ist, wobei die Tüllen (34, 35; 36, 37) ausfahrbar sind und in dem ausgerichteten Zustand einer aufgesetzten Filtervorrichtung (20) dichtend in deren Anschlüsse (8 - 11) oder Stutzen eingreifen.

2. Filtervorrichtung zur Verwendung in einem System nach Anspruch 1 mit einem länglichen Gehäuse (1)
mit zwei durch Membranen voneinander getrennten Strömungsräumen mit mindestens einem radial verlaufenden Anschluss (8 - 11) oder Anschlussstutzen, vorzugsweise zwei Anschlüssen oder Anschlussstutzen, für jeden Strömungsraum, wobei die Anschlüsse (8 - 11) auf einer Seite des Gehäuses (1) angeordnet sind und deren Mittellinien parallel zueinander verlaufen,
**dadurch gekennzeichnet,**
**dass** seitlich der Anschlüsse (8 - 11) mit einem Anschlag (33) versehene Gegenführungsstücke angeordnet sind, geeignet zur Ausrichtung der Filtervorrichtung in Längs- und Querrichtung sowie in der Tiefe in Führungen (26, 27) eines Halteteils (21), wobei die Gegenführungsstücke nach außen ragende, zueinander parallele Rippen (14, 15) umfassen.

3. Filtervorrichtung nach Anspruch 2, **dadurch gekennzeichnet, dass** die getrennten Strömungsräume durch ein in dem Gehäuse (1) gehaltertes Hohlfaserbündel gebildet werden, bei dem die Faserhohlräume den einen und der das Faserbündel umgebende Raum den anderen Strömungsraum bilden.

4. Filtervorrichtung nach Anspruch 2 oder 3, **dadurch gekennzeichnet, dass** die Anschlüsse (8 - 11) rohrabschnittförmige Mündungen aufweisen.

5. Filtervorrichtung nach Anspruch 2 bis 4, **dadurch gekennzeichnet, dass** die Mündungen der Anschlüsse (8-11) in zueinander parallelen Ebenen liegen.

6. Filtervorrichtung nach einem der Ansprüche 2 bis 5, **dadurch gekennzeichnet, dass** die Mündungen der Anschlüsse (8 - 11) in einer gemeinsamen Ebene liegen.

7. Filtervorrichtung nach Anspruch 2 bis 6, **dadurch gekennzeichnet, dass** die Mittellinien der Anschlüsse (8 - 11) auf einer gemeinsamen Ebene liegen.

8. Filtervorrichtung nach einem Ansprüche 2 bis 6, **dadurch gekennzeichnet, dass** die Mittellinien der Anschlüsse (8 - 11) auf zueinander parallelen Ebenen liegen.

9. Filtervorrichtung nach einem der Ansprüche 2 bis 8, **dadurch gekennzeichnet, dass** die Filtervorrichtung Endkappen (5) aufweist und dass sich die Anschlüsse (8 - 11) an den Endkappen (5) befinden.

10. Filtervorrichtung nach einem der Ansprüche 2 bis 9, **dadurch gekennzeichnet, dass** die Filtervorrichtung Endkappen (5) aufweist, die becherförmig ausgeführt sind und die mit jeweils einer radial über deren Böden (6) verlaufenden Wulst (7) versehen sind, in deren die Kappenränder (5) überragenden Enden die Anschlüsse (8 - 11) münden.

11. Vorrichtung zur Halterung einer Filtervorrichtung gemäß einem der Ansprüche 2 bis 10, mit Tüllen (34, 35; 36, 37), die mit den Anschlüssen (8-11) oder Anschlussstutzen der Filtervorrichtung in dichtenden Eingriff zu bringen sind, sowie mit einer Einrichtung, mit der sich die Filtervorrichtung in einer Stellung zu der Vorrichtung zur Halterung positionieren lässt, in der deren Anschlüsse (8 - 11) oder Anschlussstutzen fluchtend zu den Tüllen (34, 35; 36, 37) der Vorrichtung zur Halterung ausgerichtet sind, wobei die Einrichtung mit Führungsmitteln versehen ist, die ein anschließendes Zusammenfügen der Anschlüsse (8 - 11) und Anschlussstutzen mit den Tüllen (34, 35; 36, 37) in der Weise gewährleisten, dass diese in dichtenden Eingriff miteinander kommen, wobei ein längliches rahmen- oder gehäuseförmiges Halteteil (21), das mit Führungen (26, 27) zur Aufnahme von Gegenführungsstücken der Filtervorrichtungen (20) zu deren Ausrichtung in deren Querrichtung und in deren Tiefe und mit einem Anschlag (32) zu deren Ausrichtung in deren Längsrichtung versehen ist, wobei die Tüllen (34, 35; 36, 37) ausfahrbar sind und in dem ausgerichteten Zustand einer aufgesetzten Filtervorrichtung (20) dichtend in deren Anschlüsse (8-11) oder Stutzen eingreifen.

12. Vorrichtung nach Anspruch 11, **dadurch gekennzeichnet, dass** die Führungen aus Nuten (26, 27) bestehen, deren Flanken mit zu deren Rändern parallelen Nuten (28, 29) versehen sind, in die zueinander parallele Rippen (14, 15) greifen, die als Gegenführungsstücke seitlich der Anschlüsse oder Stutzen (8 - 11) der Filtervorrichtung (20) angeordnet sind.

13. Vorrichtung nach Anspruch 11 oder 12, **dadurch gekennzeichnet, dass** das Halteteil (21) in unterschiedlicher vertikaler Position angeordnete Nuten (26, 27) aufweist und dass die Flanken oder Ränder der oberen Nut (26) mit dem Anschlag (32) versehen sind.

14. Vorrichtung nach einem der Ansprüche 12 bis 13, **dadurch gekennzeichnet, dass** die Tüllen (34, 35; 36, 36) an einem Gleitstück (40, 50) angeordnet sind, das in Richtung von deren Längsachsen in dem Halteteil (21) verschieblich geführt ist, dass das Gleitstück (40, 50) in Richtung auf die aufgesetzte Filtervorrichtung durch mindestens ein federndes Element belastet ist und dass eine das federnde Element spannende Einrichtung vorgesehen ist, durch deren Lösen die Tüllen schnappend in die Anschlüsse oder Stutzen der aufgesetzten Filtervorrichtung eingefahren werden.

15. Vorrichtung nach einem Ansprüche 11 bis 14, **dadurch gekennzeichnet, dass** die das federnde Element spannende Einrichtung aus einem Kniehebel oder einem Exzenter besteht, der in seiner Spannstellung in seine Totpunktstellung bewegt worden ist.

16. Vorrichtung nach einem der Ansprüche 11 bis 15, **dadurch gekennzeichnet, dass** das die Tüllen tragende Gleitstück (50) einer Seite in einem schlittenartigen Klotz (56) gelagert ist, der zur Anpassung an unterschiedliche Abstände der endseitigen Anschlüsse oder Stutzen (8 - 11) der Filtervorrichtung in dem Halteteil (21) begrenzt verschieblich geführt ist.

17. Vorrichtung nach einem der Ansprüche 11 bis 16, **dadurch gekennzeichnet, dass** das Gleitstück (50) zur Ausrichtung des schlittenartigen Klotzes (56) mit einer Positioniereinrichtung (65) versehen ist.

18. Vorrichtung nach Anspruch 17, **dadurch gekennzeichnet, dass** die Positioniereinrichtung aus einem Zapfen (65) mit einer Schrägfläche (67) besteht, die an einer Anschlagkante der Filtervorrichtung (20) angreift.

## Claims (Claims for the following Contracting State(s): A, BE, CH, ES, NL)

1. A system comprising
a filter apparatus having an elongate housing (1) with two flow chambers separated from each other by membranes, with at least one radially attending connection (8-11) or connecting portion, preferably two connections or connecting portions, for each flow chamber wherein The connections (8-11) are arranged on one side of the housing (1) and the centra lines thereof extend in mutually parallel relationship, wherein arranged laterally of the connections (8-11) are counterpart guide portions provided with an abutment (33), suitable for orientation of the filter apparatus in the longitudinal and transverse directions and in depth in guides (26, 27) of a holding portion (21), wherein the counterpart guide portions include outwardly projecting mutually parallel ribs (14, 15), and
an apparatus for holding the filter apparatus, wherein the holding apparatus has nozzles (34,35; 36,37) which are to be brought into sealing engagement with the connections (8-11) or connecting portions of the filter apparatus, and there is provided a device with which the filter apparatus can be positioned in a position relative to the holder, in which the connections (8-11) or connecting portions thereof are oriented in aligned relationship with the nozzles (34, 35; 36, 37) of the holder, wherein the device is provided with guides (26, 27) for receiving counterpart guide portions (15) of the filter apparatuses (20) for orientation thereof in the transverse direction thereof and in the depth thereof and with an abutment (32) for the orientation thereof in the longitudinal direction thereof, which ensure subsequent assembly of the connections (8-11) and connecting portions with the nozzles (34, 35; 36, 37) in such a way that they come into sealing engagement with each other.

2. A filter apparatus for use in a system according to claim 1 having an elongate housing (1)
with two flow chambers separated from each other by membranes, with at least one radially extending connection (8-11) or connecting portion, preferably two connections or connecting portions, for each flow chamber, wherein the connections (9-11) are arranged on one side of the housing (1) and the centre lines thereof extend in mutually parallel relationship,
**characterised in that**
arranged laterally of the connections (8-11) are counterpart guide portions provided with an abutment (33), suitable for orientation of the filter apparatus in the longitudinal and transverse directions and in depth in guides (26, 27) of a holding portion (21), wherein the counterpart guide portions include outwardly projecting, mutually parallel ribs (14, 15).

3. A filter apparatus according to claim 2 **characterised in that** the separated flow chambers are formed by a hollow fibre bundle which is held in the housing (1), in which the fibre hollow spaces from the one flow chamber and the space surrounding the fibre bundle forms the other flow chamber.

4. A filter apparatus according to claim 2 or claim 3 **characterised in that** the connections (8-11) have mouth openings in the form of tube portions.

5. A filter apparatus according to claims 2 to 4 **characterised in that** the mouth openings of the connections (8-11) are in mutually parallel planes.

6. A filter apparatus according to one of claims 1 to 5 **characterised in that** the mouth openings of the connections (8-11) lie in a commun plane.

7. A filter apparatus according to one of claims 1 to 6 **characterised in that** the centre lines of the connections (8-11) lie on a common plane.

8. A filter apparatus according to one of claims 1 to 6 **characterised in that** the centre lines of the connections (8-11) lie on mutually parallel planes.

9. A filter apparatus according to one of claims 1 to 8 **characterised in that** the filter apparatus has end caps (5) and that the connections (8-11) are on the end caps (5).

10. A filter apparatus according to one of claims 1 to 9 **characterised in that** the filter apparatus has end caps (5) which are of a cup-shaped configuration and which are each provided with a respective ridge (7) extending radially over the end portions (6) thereof, the connections (8-11) communicating with the ridge ends which project beyond the cap edges (5).

11. Apparatus for holding a filter apparatus according to one of claims 1 to 10 **characterised in that** the holding apparatus has nozzles (34, 35; 36, 37) which are to be brought into sealing engagement with the connections (8-11) or connecting portions of the filter apparatus, and that there is provided a device with which the filter apparatus can be positioned in a position relative to the holder, in which the connections (8-11) or connecting portions thereof are oriented in aligned relationship with the nozzles (34, 35; 36, 37) of the holder, wherein the device is provided with guides (26, 27) for receiving counterpart guide portions (15) of the filter apparatuses (20) for orientation thereof in the transverse direction thereof and in the depth thereof and with an abutment (32) for the orientation thereof in the longitudinal direction thereof, which ensure subsequent assembly of the connections (8-11) and connecting portions with the nozzles (34,35; 36,37) in such a way that they come into sealing engagement with each other.

12. Apparatus according to claim 11 **characterised in that** the guides (26, 27) are arranged in an elongate holding portion (21) in the form of a frame or housing and that the nozzles (34, 35; 36, 37) are extendable and in the oriented condition of a fitted filter apparatus (20) engage sealingly into the connections (8-11) or connecting portions thereof.

13. Apparatus according to claim 12 **characterised in that** the guides comprise grooves (26, 27) whose flanks are provided with grooves (28, 29) which are parallel to the edges thereof and into which engage mutually parallel ribs (14, 15) which are arranged as counterpart guide portions laterally of the connections or connecting portions (8-11) of the filter apparatus (20).

14. Apparatus according to claim 12 or claim 13 **characterised in that** the holding potion (21) has grooves (26, 27) arranged in different vertical positions and that the flanks or edges of the upper groove (26) are provided with an abutment (32).

15. Apparatus according to one of claims 12 to 14 **characterised in that** the nozzles (34, 35; 36, 36) are arranged on a slide portion (40, 50) which is guided displaceably in the direction of the longitudinal axes thereof in the holding portion (21), that the slide portion (40, 50) is loaded in a direction towards the fitted filter apparatus by at least one resilient element and that there is provided a device for stressing the resilient element and by the release of which the nozzles are snappingly moved into the connections or connecting portions of the fitted filter apparatus.

16. Apparatus according to one of claims 12 to 15 **characterised in that** the device for stressing the resilient element comprises an elbow lever or an eccentric which in its stressing positon has been moved into its dead centre position.

17. Apparatus according to one of claims 12 to 16 **characterised in that** the slide portion (50) carrying the nozzles of one side is mounted in a slide-like block (56) vvhich is limitedly displaceably guided in the holding portion (21) for adaptation to different spacings of the end connections or connecting portions (8-11) of the filter apparatus.

18. Apparatus according to one of claims 12 to 17 **characterised in that** the slide portion (50) is provided with a positioning device (65) for orientation of the slide-like black (56).

19. Apparatus according to claim 18 **characterised in that** the positioning device 19. Apparatus according to claim 18 **characterised in that** the positioning device comprises a projection (65) with an inclined surface (67) which engages against an abutment edge of the filter apparatus (20).

## Claims (Claims for the following Contracting State(s): A, BE, CH, ES, NL)

1. A system comprising
a filter apparatus having an elongate housing (1) with two flow chambers separated from each other by membranes, with at least one radially extending connection (8-11) or connecting portion, preferably two connections or connecting portions, for each flow chamber, wherein the connections (8-11) are arranged on one side of the housing (1) and the centre lines thereof extend in mutually parallel relationship, wherein arranged laterally of the connections (8-11) are counterpart guide portions provided with an abutment (33), suitable for orientation of the filter apparatus in the longitudinal and transverse directions and in depth in guides (26, 27) of a holding portion (21), wherein the counterpart guide portions include outwardly projecting, mutually parallel ribs (14, 15), and
an apparatus for holding the filter apparatus, having nozzles (34, 35; 36, 37) which are to be brought into sealing engagement with the connections (8-11) or connecting portions of the filter apparatus, and with a device with which the filter apparatus can be positioned in a position relative to the holding apparatus, in which the connections (8-11) or connecting portions thereof are oriented in aligned relationship with the nozzles (34, 35; 36, 37) of the holding apparatus, wherein the device is provided with guide means which ensure subsequent assembly of the connections (8-11) and connecting portions with the nozzles (34, 35; 36, 37) in such a way that they come into sealing engagement with each other, wherein there is provided an elongate holding portion (21) in the form of a frame or housing and which is provided with guides (26, 27) for receiving counterpart guide portions (15) of the filter apparatuses (20) for orientation thereof in the transverse direction thereof and in the depth thereof and with an abutment (32) for the orientation thereof in the longitudinal direction thereof, wherein the nozzles (34, 35; 36, 37) are extendable and in the oriented condition of a fitted filter apparatus (20) engage sealingly into the connections (8-11) or connecting portions thereof.

2. A filter apparatus for use in a system according to claim 1 having an elongate housing (1)
with two flow chambers separated from each other by membranes, with at least one radially extending connection (8-11) or connecting portion, preferably two connections or connecting portions, for each flow chamber, wherein the connections (8-11) are arranged on one side of the housing (1) and the centre lines thereof extend in mutually parallel relationship,
**characterised in that**
arranged laterally of the connections (8-11) are counterpart guide portions provided with an abutment (33), suitable for orientation of the filter apparatus in the longitudinal and transverse directions and in depth in guides (26, 27) of a holding portion (21), wherein the counterpart guide portions include outwardly projecting, mutually parallel ribs (14,15).

3. A filter apparatus according to claim 2 **characterised in that** the separated flow chambers are formed by a hollow fibre bundle which is held in the housing (1), in which the fibre hollow spaces form the one flow chamber and the space surrounding the fibre bundle forms the other flow chamber.

4. A filter apparatus according to claim 2 or claim 3 **characterised in that** the connections (8-11) have mouth openings in the form of tube portions.

5. A filter apparatus according to claims 2 to 4 **characterised in that** the mouth openings of the connections (8-11) are in mutually parallel planes.

6. A filter apparatus according to one of claims 2 to 5 **characterised in that** the mouth openings of the connections (8-11) lie in a common plane.

7. A filter apparatus according to one of claims 2 to 6 **characterised in that** the centre lines of the connections (8-11) lie on a common plane.

8. A filter apparatus according to one of claims 2 to 6 **characterised in that** the centre lines of the connections (8-11) lie on mutually parallel planes.

9. A filter apparatus according to one of claims 2 to 8 **characterised in that** the filter apparatus has end caps (5) and that the connections (8-11) are on the end caps (5).

10. A filter apparatus according to one of claims 2 to 9 **characterised in that** the filter apparatus has end caps (5) which are of a cup-shaped configuration and which are each proved with a respective ridge (7) extending radially over the end portions (6) thereof, the connections (8-11) communicating with the ridge ends which project beyond the cap edges (5).

11. Apparatus for holding a filter apparatus according to one of claims 2 to 10, with nozzles (34, 35; 36, 37) which are to be brought into sealing engagement with the connections (8-11) or connecting portions of the filter apparatus, and with a device with which the filter apparatus can be positioned in a position relative to the holding apparatus, in which the connections (8-11) or connecting portions thereof are oriented in aligned relationship with the nozzles (34, 35; 36, 37) of the holding apparatus, wherein the device is provided with guide means which ensure subsequent assembly of the connections (8-11) and connecting portions with the nozzles (34, 35; 36, 37) in such a way that they come into sealing engagement with each other, wherein there is provided an elongate holding portion (21) in the form of a frame or housing, which is provided with guides (26, 27) for receiving counterpart guide portions (15) of the filter apparatuses (20) for orientation thereof in the transverse direction thereof and in the depth thereof and with an abutment (32) for the orientation thereof in the longitudinal direction thereof, wherein the nozzles (34, 35; 36, 37) are extendable and in the oriented condition of a fitted filter apparatus (20) engage sealingly into the connections (8-11) or connecting portions thereof.

12. Apparatus according to claim 11 **characterised in that** the guides comprise grooves (26,27) whose flanks are provided with grooves (28, 29) which are parallel to the edges thereof and into which engage mutually parallel ribs (14, 15) which are arranged as counterpart guide portions laterally of the connections or connecting portions (8-11) of the filter apparatus (20).

13. Apparatus according to claim 11 or claim 12 **characterised in that** the holding portion (21) has grooves (26, 27) arranged in different vertical positions and that the flanks or edges of the upper groove (26) are provided with an abutment (32).

14. Apparatus according to one of claims 12 and 13 **characterised in that** the nozzles (34, 35; 36, 36) are arranged on a slide portion (40, 50) which is guided displaceably in the direction of the longitudinal axes thereof in the holding portion (21), that the slide portion (40, 50) is loaded in a direction towards the fitted filter apparatus by at least one resilient element and that there is provided a device for stressing the resilient element and by the release of which the nozzles are snappingly moved into the connections or connecting portions of the filter apparatus.

15. Apparatus according to one of claims 11 to 14 **characterised in that** the device for stressing the resilient element comprises an elbow lever or an eccentric which in its stressing portion has been moved into its dead centre position.

16. Apparatus according to one of claims 11 to 15 **characterised in that** the slide portion (50) carrying the nozzles of one side is mounted in a slide-like block (56) which is limitedly displaceably guided in the holding portion (21) for adaptation to different spacings of the end connections or connecting portions (8-11) of the filter apparatus.

17. Apparatus according to one of claims 11 to 16 **characterised in that** the slide portion (50) is provided with a positioning device (65) for orientation of the slide-like block (56).

18. Apparatus according to claim 17 **characterised in that** the positioning device comprises a projection (65) with an inclined surface (67) which engages against an abutment edge of the filter apparatus (20).

## Revendications (Revendications pour l'(les) Etat(s) contractant(s) suivant(s): DE, FR, GB, IT)

1. Système comprenant
un dispositif de filtrage avec un boîtier (1) allongé avec deux compartiments d'écoulement séparés l'un de l'autre par des membranes avec au moins un raccordement (8 11) ou une tubulure de raccordement agencée radialement, de préférence deux raccordements ou tubulures de raccordement, pour chaque compartiment d'écoulement, les raccordements (8 - 11) étant disposés sur un côté du boîtier (1) et leurs lignes médianes étant parallèles entre elles, des pièces de contre-guidage pourvues d'une butée (33) étant disposées sur le côté des raccordements (8 - 11), appropriées pour l'orientation du dispositif de filtrage dans le sens longitudinal et le sans transversal ainsi que dans la profondeur dans des guides (26, 27) d'une pièce de retenue (21), les pièces de contre-guidage comprenant des nervures (14, 15) débordant vers l'extérieur et parallèles entre elles, et
un dispositif pour le support de ce dispositif de filtrage, où le dispositif pour le support présente des douilles (34, 35, 36, 37), qui doivent être mises en prise pour l'étanchéité avec les raccordements (8 - 11) ou les tubulures de raccordement du dispositif de filtrage, et en ce qu' un système est prévu avec lequel le dispositif de filtrage peut être positionné dans une position par rapport au dispositif de support, dans laquelle ses raccordements (8 - 11) ou tubulures de raccordement sont orientes en alignement avec les douilles (34, 35; 36, 37) du dispositif de support, le système étant pourvu de guidages (26, 27) pour la réception de pièces de contre-guidage (14, 15) des dispositifs de filtrage (20) pour leur orientation dans leur direction transversale et dans leur profondeur et d'une butée (32) pour leur orientation dans leur direction longitudinale, qui assurent un assemblage consécutif des raccordements (B - 11) et tubulures de raccordement avec les douilles (34, 35; 36, 37) en ce sens que celles-ci viennent en prise étanche les unes avec les autres.

2. Dispositif de filtrage pour la réalisation dans un système selon la revendication 1 avec un boîtier (1) allongé
avec deux compartiments d'écoulement séparés l'un de l'autre par des membranes avec au moins un raccordement (8 11) ou tubulure de raccordement agencée radialement, de préférence deux raccordements ou tubulures de raccordement, pour chaque compartiment d'écoulement, les raccordements (8 - 11) étant disposés sur un côté du boîtier (1) et dont les lignes médianes sont parallèles entre elles,
**caractérisé en ce que**
des pièces de contre-guidage pourvues d'une butée (33) sont disposées sur le côté des raccordements (8 - 11), appropriées pour l'orientation du dispositif de filtrage dans le sens longitudinal et le sens transversal ainsi que dans la profondeur dans des guides (26, 27) d'une partie de retenue (21), les pièces de contre-guidage comprenant des nervures (14, 15) dépassant vers l'extérieur et parallèles entre elles.

3. Dispositif de filtrage selon la revendication 2, **caractérisé en ce que** les compartiments d'écoulement séparés sont formés par un faisceau de fibres creuses maintenu dans le boîtier (1), dans lequel les cavités de fibre forment un compartiment d'écoulement et le compartiment entourant le faisceau de fibres l'autre compartiment d'écoulement.

4. Dispositif de filtrage selon la revendication 2 ou 3, **caractérisé en ce que** les raccordements (8 - 11) présentent des orifices en forme de section de tube.

5. Dispositif de filtrage selon les revendication 2 à 4, **caractérisé en ce que** les orifices des raccordements (8 - 11) sont disposés dans des plans parallèles entre eux.

6. Dispositif de filtrage selon l'une des revendications 1 à 5, **caractérisé en ce que** les orifices des raccordements (8 - 11) sont disposés dans un plan commun.

7. Dispositif de filtrage selon l'une des revendications 1 à 6, **caractérisé en ce que** les lignes médianes des raccordements (8 - 11) sont disposées sur un plan commun.

8. Dispositif de filtrage selon l'une des revendications 1 à 6, **caractérisé en ce que** les lignes médianes des raccordements (B - 11) sont situées sur des plans parallèles entre eux.

9. Dispositif de filtrage selon l'une des revendications 1 à 8, **caractérisé en ce que** les dispositifs de filtrage présentent des capuchons d'extrémité (5) et **en ce que** les raccordements (8 - 11) se trouvent sur les capuchons d'extrémité (5).

10. Dispositif de filtrage selon l'une des revendications 1 à 9, **caractérisé en ce que** le dispositif de filtrage présente des capuchons d'extrémité (5), qui sont conçus en forme de gobelet et qui sont pourvus de respectivement un renflement (7) agencé radialement sur leurs fonds (6), les raccordements (8 - 11) débouchant dans les extrémités, dépassant des bords de capuchon (5), du renflement.

11. Dispositif pour le support d'un dispositif de filtrage selon l'une des revendications 1 à 10, **caractérise en ce que** le dispositif de support comprend des douilles (34, 35, 36, 37), qui doivent être mises en prise pour l'étanchéité avec les raccordements (8 - 11) ou les tubulures de raccordement du dispositif de filtrage, et **en ce qu'**un système est prévu avec lequel le dispositif de filtrage peut être positionné dans une position par rapport au dispositif de support, dans laquelle ses raccordements (8 - 11) ou tubulures de raccordement sont orientée en alignement avec les douilles (34, 35; 36, 37) du dispositif de support, le système étant pourvu de guidages (26, 37) pour la réception de pièces de contre-guidage (14, 15) des dispositifs de filtrage (20) pour leur orientation dans leur direction transversale et dans leur profondeur et d'une butée (32) pour leur orientation dans leur direction longitudinale, qui assurent un assemblage consécutif des raccordements (8 - 11) et tubulures de raccordement avec les douilles (34, 35; 36, 37) en ce sens que celles-ci viennent en prise étanche les unes avec les autres.

12. Dispositif selon la revendication 11, **caractérisé en ce que** les guidages (26, 27) sont disposés dans une partie de retenue oblongue (21) en forme de cadre et boîtier, et **en ce que** les douilles (34, 35; 36, 37) peuvent être sorties et, à l'état oriente d'un dispositif de filtrage appliqué (20), s'engagent d'une manière étanche dans les raccordements (8-11) ou tubulures.

13. Dispositif selon la revendication 12, **caractérisé en ce que** les guidages se composent de rainures (26, 27), dont les flancs sont dotés de rainures (28, 29) parallèles à leurs bords, dans lesquelles s'engagent des nervures (14, 15) parallèles entre elles, qui sont disposées comme pièces de contre-guidage sur le côté des raccordements ou tubulures (8 - 11) du dispositif de filtrage (20).

14. Dispositif selon la revendication 12 ou 13, **caractérisé en ce que** la partie de retenue (21) présente des rainures (26, 27) disposées dans une position verticale différente et **en ce que** les flancs au bords de la rainure (26) supérieure sont dotés de la butée (32).

15. Dispositif selon l'une des revendications 12 à 14, **caractérisé en ce que** les douilles (34, 35; 36, 37) sont disposées sur une pièce coulissante (40, 50), qui est guidée de façon coulissante en direction de leurs axes longitudinaux dans la partie de retenue (21), que la pièce coulissante (40, 50) est sollicitée en direction du dispositif de filtrage posé dessus par au moins un élément élastique et **en ce qu'**il est prévu un dispositif tendant l'élément élastique, par le desserrage duquel les douilles sont engagées par encliquetage dans les raccordements ou tubulures du dispositif de filtrage posé dessus.

16. Dispositif selon l'une des revendications 12 à 15, **caractérisé en ce que** le dispositif tendant l'élément élastique sa compose d'un levier coudé ou d'un excentrique, qui a été déplacé dans sa position de serrage à sa position de point mort.

17. Dispositif selon l'une des revendications 12 à 16, **caractérisé en ce que** la pièce coulissante (50), portant les douilles, d'un côté est logée dans un bloc (56) en forme de coulisseau, qui est guide dans la partie de retenue (21) de façon coulissante et limitée pour l'adaptation à différents espacements des raccordements ou tubulures (8 - 11) côté extrémité du dispositif de filtrage.

18. Dispositif selon l' une des revendications 12 à 17, **caractérisé en ce que** la pièce coulissante (50) est dotée d'un système de positionnement (65) pour l'orientation du bloc (56) en forme de coulisseau.

19. Dispositif selon la revendication 18, **caractérisé en ce que** le système de positionnement se compose d' un tenon (65) avec une surface inclinée (67) qui s'applique à un bord de butée du dispositif de filtrage (20).

## Revendications (Revendications pour l'(les) Etat(s) contractant(s) suivant(s): DE, FR, GB, IT)

1. Système comprenant
un dispositif de filtrage avec un boîtier (1) allongé avec deux compartiments d'écoulement séparés l'un de l'autre par des membranes avec au moins un raccordement (8 11) ou une tubulure de raccordement agencée radialement, de préférence deux raccordements ou tubulures de raccordement, pour chaque compartiment d'écoulement, les raccordements (8 - 11) étant disposés sur un côte du boîtier (1) et leurs lignes médianes étant parallèles entre elles, des pièces de contre-guidage pourvues d'une butée (33) étant disposées sur le côté des raccordements (8 - 11), appropriées pour l'orientation du dispositif de filtrage dans le sens longitudinal et le sens transversal ainsi que dans la profondeur dans des guides (26, 27) d'une pièce de retenue (21), les pièces de contre-guidage comprenant des nervures (14, 15) débordant vers l'extérieur et parallèles entre elles, et
un dispositif pour le support de ce dispositif de filtrage, avec des douilles (34, 35; 36, 37), qui doivent être mises en prise pour l'étanchéité avec les raccordements (8 - 11) ou les tubulures de raccordement du dispositif de filtrage, et avec un système avec lequel le dispositif de filtrage peut être positionné dans une position par rapport au dispositif de support, dans lequel ses raccordements (8 - 11) ou tubulures de raccordement sont orientés en alignement avec les douilles (34, 35; 36, 37) du dispositif pour le support, le système étant pourvu de moyens de guidage qui garantissent un assemblage consécutif des raccordements (8 - 11) et tubulures de raccordement avec les douilles (34, 35; 36, 37) en ce sens que celles-ci viennent en prise pour l'étanchéité les unes avec les autres, une partie de retenue (21) allongée et en forme de cadre ou de boîtier, qui est pourvue de guides (26, 27) pour le logement de pièces de contre-guidage des dispositifs de filtrage (20) pour leur alignement dans leur sens transversal et dans leur profondeur et d'une butée (32) pour leur orientation dans leur sens longitudinal, les douilles (34, 35; 36, 37) pouvant être sorties et s'engageant, lorsqu'un dispositif de filtrage (20) posé dessus est orienté de façon étanche dans ses raccordements (8 - 11) ou tubulures.

2. Dispositif de filtrage pour la réalisation dans un Système selon la revendication 1 avec un boîtier (1) allongé
avec deux compartiments d'écoulement séparés l'un de l'autre par des membranes avec au moins un raccordement (8 11) ou tubulure de raccordement agencée radialement, de préférence deux raccordements ou tubulures de raccordement, pour chaque compartiment d'écoulement, les raccordements (8 - 11) étant disposés sur un côté du boîtier (1) et dont les lignes médianes sont parallèles entre elles,
**caractérisé en ce que**
des pièces de contre-guidage pourvues d'une butée (33) sont disposées sur le côté des raccordements (8 - 11), appropriées pour l'orientation du dispositif de filtrage dans le sens longitudinal et le sens transversal ainsi que dans la profondeur dans des guides (26, 27) d'une partie de retenue (21), les pièces de contre-guidage comprenant des nervures (14, 15) dépassant vers l'extérieur et parallèles entre elles.

3. Dispositif de filtrage selon la revendication 2, **caractérisé en ce que** les compartiments d'écoulement séparés sont formés par un faisceau de fibres creuses maintenu dans le boîtier (1), dans lequel les cavités de fibre forment un compartiment d'écoulement et le compartiment entourant le faisceau de fibres l'autre compartiment d'écoulement.

4. Dispositif de filtrage selon la revendication 2 ou 3, **caractérisé en ce que** les raccordements (8 - 11) présentent des orifices en forme de section de tube.

5. Dispositif de filtrage selon les revendications 2 à 4, **caractérisé en ce que** les orifices des raccordements (8 11) sont disposés dans des plans parallèles entre eux.

6. Dispositif de filtrage selon l'une quelconque des revendications 2 à 5, **caractérisé en ce que** les orifices des raccordements (8 - 11) sont disposés dans un plan commun.

7. Dispositif de filtrage selon les revendications 2 à 6, **caractérisé en ce que** les lignes médianes des raccordements (8 - 11) sont disposées sur un plan commun.

8. Dispositif de filtrage selon l'une quelconque des revendications 2 à 6, **caractérisé en ce que** les lignes médianes des raccordements (8 - 11) sont situées sur des plans parallèles entre eux.

9. Dispositif de filtrage selon l'une quelconque des revendications 2 à 8, **caractérisé en ce que** les dispositifs de filtrage présentent des capuchons d'extrémité (5) et **en ce que** les raccordements (8 - 11) se trouvent sur les capuchons d'extrémité (5).

10. Dispositif de filtrage selon l'une quelconque des revendications 2 à 9, **caractérisé en ce que** le dispositif de filtrage présente des capuchons d'extrémité (5), qui sont conçus en forme de gobelet et qui sont pourvus de respectivement un renflement (7) agencé radialement sur leurs fonds (6), les raccordements (8 - 11) débouchant dans les extrémités, dépassant des bords de capuchon (5), du renflement.

11. Dispositif pour le support d'un dispositif de filtrage selon l'une quelconque des revendications 2 à 10, comprenant des douilles (34, 35; 36, 37) qui peuvent être mises en prise pour l'étanchéité avec les raccordements (8 - 11) ou les tubulures de raccordement du dispositif de filtrage, et un système avec lequel le dispositif de filtrage peut être positionné dans une position par rapport au dispositif de support, dans lequel ses raccordements (8 - 11) ou tubulures de raccordement sont orientés en alignement avec les douilles (34, 35; 36, 37) du dispositif de support, le système étant pourvu de moyens de guidage qui garantissent un assemblage consécutif des raccordements (8 - 11) et tubulures de raccordement avec les douilles (34, 35; 36, 37) en ce sens que celles-ci viennent en prise d'étanchéité entre elles, une partie de retenue (21) allongée, en forme de cadre ou de boîtier, qui est pourvue de guides (26, 27) pour le logement de pièces de contre-guidage du dispositif de filtrage (20) pour leur orientation dans leur sens transversal et dans leur profondeur et d'une butée (32) pour leur orientation dans leur sens longitudinal, les douilles (34, 35; 36, 37) prouvant être sorties et s'engageant, lorsque le dispositif de filtrage (20) posé dessus est orienté, de façon étanche dans leurs raccordements (8 - 11) ou tubulures.

12. Dispositif selon la revendication 11, **caractérisé en ce que** les guides se composent de rainures (26, 27), dont les flancs sont dotés de rainures (28, 29) parallèles à leurs bords, dans lesquelles s'engagent des nervures (14, 15) parallèles entre elles, qui sont disposées comme pièces de contre-guidage sur le côté des raccordements ou tubulures (8 - 11) du dispositif de filtrage (20).

13. Dispositif selon la revendication 11 ou 12, **caractérisé en ce que** la partie de retenue (21) présente des rainures (26, 27) disposées dans une position verticale différente et **en ce que** les flancs ou bords de la rainure (26) supérieure sont dotés de la butée (32).

14. Dispositif selon l'une quelconque des revendications 12 à 13, **caractérisé en ce que** les douilles (34, 35; 36, 37) sont disposées sur une pièce coulissante (40, 50), qui est guidée de façon coulissante en direction de leurs axes longitudinaux dans la partie de retenue (21), que la pièce coulissante (40, 50) est sollicitée en direction du dispositif de filtrage posé dessus par au moins un élément élastique et **en ce qu'**il est prévu un Dispositif tendant l'élément élastique, par le desserrage duquel les douilles sont engagées par encliquetage dans les raccordements ou tubulures du dispositif de filtrage posé dessus.

15. Dispositif selon l'une quelconque des revendications 11 à 14, **caractérisé en ce que** le dispositif pendant l'élément élastique se compose d'un levier coudé ou d'un excentrique, qui a été déplacé dans sa position de serrage à sa position de point mort.

16. Dispositif selon l'une quelconque des revendications 11 à 15, **caractérisé en ce que** la pièce coulissante (50), portant les douilles, d'un côté est logée dans un bloc (56) en forme de coulisseau, qui est guidé dans la partie de retenue (21) de façon coulissante et limitée pour l'adaptation à différents espacements des raccordements ou tubulures (8 - 11) côté extrémité du dispositif de filtrage.

17. Dispositif de filtrage selon l'une quelconque des revendications 11 à 16, **caractérisé en ce que** la pièce coulissante (50) est dotée d'un système de positionnement (65) pour l'orientation du bloc (56) en forme de coulisseau.

18. Dispositif selon la revendication 17, **caractérisé en ce que** le système de positionnement se compose d'un tenon (65) avec une surface inclinée (67) qui s'applique sur un bord de butée du dispositif de filtrage (20).
